# EUROPEAN PATENT APPLICATION

(11) **EP 1 683 537 A1**
(43) Date of publication of application: **26.07.2006**
(21) Application number: 05100448.9
(22) Date of filing: 25.01.2005
(51) Int. Cl.: A61M 5/315, A61M 5/24

(54) **Injection device for administering a medication liquid**

(71) Applicant: Pfizer Health AB, 112 87 Stockholm (SE)
(72) Inventor: The designation of the inventor has not yet been filed
(74) Representative: Courgeon, Antoine

(57) **Abstract**

Multiple dose disposable injection device (1) for reconstituting a medication liquid before its injection and for permanently adjusting the volume of the doses of medication liquid to be administered. The injection device (1) is additionally totally undemountable after the final use.

## Description

The present invention relates to an injection device for administering multiple doses of a medication liquid.

This device is aimed more particularly at a syringe-type injection device for administering to oneself or to another person multiple doses of a medication liquid taken from an cartridge.

### Background of the invention

Devices of the above kind are already known in the art, in particular for administering doses of insulin to patients suffering from diabetes. They generally comprise a housing adapted to be held by the user and to a distal end of which is fixed an cartridge fitted with a needle and containing the prescribed dose of insulin. A movable wall actuated by the patient moves longitudinally inside the housing to expel the dose of insulin from the cartridge and inject it via the needle. Thus a patient can usually administer to himself the daily dose of insulin that he requires, without the assistance of another person.

In the case of insulin, the problem of reconstituting the medication to be administered does not arise because insulin is sold ready for use. The same does not apply to growth hormones, for example, which may take the form of a powder constituting the active ingredient and which must be mixed with a solvent prior to use. In the powder state, growth hormones have a shelf life of the order of some years, whereas, once mixed with the solvent, the resulting product has a shelf life of no more than thirty days, whence the benefit of storing growth hormones in the powder state.

Two types of syringe for reconstituting the medication to be administered are already commercially available. The first of these two types of syringe is a single-dose disposable syringe that can therefore be used for only one injection. The second type of syringe is a re-usable multiple dose injection device. When the cartridge containing the medication liquid to be administered is empty, it may be replaced, and the syringe itself is not disposed of. As far as the Applicant is aware, there is no commercially available disposable multiple dose injection device of the kind referred to here enabling a medication to be reconstituted prior to its administration.

The ability to set the volume of the dose to be administered irreversibly may also be of benefit in relation to the nature of the treatment to be administered. This applies in particular to growth hormones, since treatment with this kind of product is always spread over a long period of time, during which the patient receives a daily injection that always contains the same quantity of medication.

One prior art system determines the volume of the dose to be injected by a number of clicks corresponding to the movement of ratchets along clipping means. This solution lacks safety features, especially if the person having to administer an injection is a child or is handicapped and has lost his manipulation faculties, or in an emergency when it is necessary to inject a predetermined dose of an active substance very quickly.

Other prior art re-usable injection devices adjust the dose to be administered more precisely but comprise a large number of component parts, which is reflected in a high unit cost.

### Summary of the Invention

An object of the present invention is to remedy the problems mentioned above, and further problems, by providing an injection device in particular enabling a medication liquid to be reconstituted before it is injected and enabling permanent setting of the volume of the doses of medication liquid to be administered. Preferably, the injection device of the invention is disposable.

Another problem which has not found a satisfactory solution as yet concerns determining the dose to be injected. The aim is to be able to set once and for all the volume of the doses to be administered successively, so that a child or a young adolescent cannot modify the dose at his own initiative, for example, with the risk of making a mistake.

The present invention also provides an injection device for administering multiple doses of a medication liquid, said device comprising a housing a cartridge containing a preparation or precursor component for the preparation and having a front part and a rear part, a stopper arranged at the front part and the rear part being closed by a movable wall, a displacement of which wall causes the preparation to be moved forward or expelled through the stopper adapted to be perforated, for the preparation, and actuating means for actuating the wall via a plunger whose forward movement inside the housing is driven by an actuator at the proximal end of the housing and adapted to be actuated by the user, the injection device further comprising preselector means for preselecting a dose to be injected.

In a first aspect, the present invention provides an injection device of the kind described above that is characterised in that it comprises means for permanently setting the dose to be administred.

In an embodiment, the invention comprises means for permanently immobilising the preselector means after setting the dose to be administered.

Thanks to the above features, one object of the present invention provides an injection device for administering multiple doses of the same volume of a medication liquid, the volume of said doses being set on the first use of the injection device and not being modifiable thereafter, during use of said device. The injection device of the invention therefore provides a simple and safe way of setting the volume of the doses to be administered, and is particularly advantageous when the device is to be used by a child, for example. It is sufficient for an adult to set the volume of the dose as a function of the medical prescription, after which the child can administer the required doses itself, without being able to modify the dose intentionally or accidentally.

According to a second aspect, the present invention provides an injection device of the kind described hereinabove that is characterised in that it comprises means for permanently fastening the actuator to the housing after administering the final dose of the medication liquid.

Thanks to these features, an other object of the present invention provides a non-demountable injection device satisfying strict safety criteria. If the volume of the doses to be administered is not an integer fraction of the total volume of the medication liquid contained in the cartridge, a residue of liquid remains in said cartridge after administering the final dose. Making the injection device of the invention undemountable prevents the user, in particular a child, from accessing the remainder of the medication liquid contained in the cartridge, and ingesting it, for example, and likewise prevents the user from administering to himself the residual liquid, which does not correspond to the prescribed volume. The user is also prevented from injuring himself or ingesting debris from the glass cartridge.

In a third aspect, the present invention provides an injection device of the kind described above that is characterised in that it further comprises a bush adapted to be moved forward and backward within the housing and having at its periphery longitudinal grooves of different lengths, a first stop member being adapted to enter any of said grooves and to define with said groove a position of maximum retraction of the bush, said bush being also adapted to be immobilised in the forward direction by a second stop member that defines with the first stop member the active travel of the bush within the housing and thereby determines the volume of the dose to be injected.

Thanks to these features, an other object of the present invention provides an injection device in which the means for setting the volume of the dose to be injected are simple and reliable, which is particularly advantageous if the user is a child or a young adolescent.

In a fourth aspect, the present invention provides an injection device of the kind described above that is characterised in that the plunger has along its length a cam surface with which co-operates at least one latching member that is latched when the plunger reaches a predefined position.

According to an additional feature of the invention, the latching member is a detent which is latched when it enters a longitudinal groove in the housing of said injection device to immobilise permanently the preselector means after setting the dose to be administered.

In a preferred embodiment,the plunger reaches the predefined position before the first injection. The skilled person would easily be able to define the best predefined position depending on when the dose to be administered should be permanently set.

According to another feature of the invention, the latching member is a detent which, when latched, is retracted in front of an immobilising member that projects into an opening in the housing of said injection device to immobilise the administration mechanism permanently after dispensing the final dose.

Thanks to these features, the present invention provides an injection device enabling permanent and automatic preselection of the dose to be injected after the first use. Furthermore, the present invention provides an injection device that is locked automatically after administering the final dose, without necessitating intervention by the user. These features make said device even safer to use.

In a fifth aspect, the present invention provides an injection device of the kind described above that is characterised in that, in a first mode of operation for reconstituting the medication liquid, the plunger is actuated by the actuator, which operates on the rear portion thereof, and in that, in a second mode of operation for dispensing the medication liquid, the actuator operates on the plunger via a system of detents and racks.

Thanks to these features, the present invention provides an injection device able to deliver doses of a medication liquid that has to be reconstituted before use. This is particularly advantageous if the substance to be administered is a growth hormone, for example. Growth hormones typically take the form of a powder that must be diluted in a solvent before administration. In the powder state, the shelf life of growth hormones is approximately some years. Once reconstituted, these products must be administered within thirty days. The benefit of storing growth hormones in the powder state and diluting them only at the time of use is clear. The injection device of the invention advantageously reconstitutes the medication to be administered as a first step and then delivers doses of the same volume of said medication as a second step.

In a sixth aspect, the present invention provides an injection device of the kind described above that is characterised in that it comprises a bush comprising first and second latching means that co-operate with a stop member to immobilise said bush against rotation and against movement in translation, respectively, the actuator and the bush being such that, on a first use, the actuator, when actuated by the user, moves forward relative to said bush, which remains fixed and releases the second latching means, with the result that the bush is free to move in translation, said actuator then being fixed to said bush to move in translation and to rotate therewith.

Thanks to these features, the present invention provides an injection device that cannot be used prior to intentional action by the user. This excludes involuntary actuation of said injection device.

### Brief description of the drawings

Other features and advantages of the present invention will emerge more clearly from the following detailed description of one purely illustrative and nonlimiting embodiment of an injection device according to the invention, given with reference the appended drawings, in which:
- figure 1 is an exploded view of an injection device of the invention;
- figures 2A to 2G are diagrammatic representations of the injection device of the invention showing the succession of operations that the user must carry out by means of the actuator;
- figure 3 is a front view of the injection device of the invention;
- figure 4 is a view in section taken along the line A-A in figure 3 of the injection device of the invention prior to its first use, i.e. prior to reconstituting the medication liquid;
- figure 5 is a view in section taken along the line B-B in figure 3 of the injection device of the invention prior to its first use, i.e. prior to reconstituting the medication liquid;
- figures 6 and 7 are views to a larger scale of the ringed areas X and Y in figure 4;
- figure 8 is a view to a larger scale of the ringed area in figure 5;
- figure 9 is a front view of the injection device of the invention;
- figure 10 is a view in section taken along the line A-A in figure 9 of the injection device of the invention ready to administer a dose of medication liquid;
- figure 11 is a view in section taken along the line D-D in figure 9 of the injection device of the invention ready to administer a dose of medication liquid;
- figure 12 is a view to a larger scale of the ringed area X in figure 10;
- figures 13, 14 and 15 are views to a larger scale of the ringed areas W, Y and Z in figure 11;
- figure 16 is a front view of the injection device of the invention;
- figure 17 is a view in section taken along the line C-C in figure 16 of the injection device of the invention after injecting a dose of medication liquid;
- figure 18 is a view in section taken along the line D-D in figure 16 of the injection device of the invention after injecting a dose of medication liquid;
- figure 19 is a view to a larger scale of the ringed area X in figure 17;
- figures 20, 21 and 22 are views to a larger scale of the ringed areas W, Y and Z in figure 18;
- figure 23 is a front view of the injection device of the invention;
- figure 24 is a view in section taken along the line B-B in figure 23 of the injection device of the invention after administering the final dose of medication liquid;
- figure 25 is a view in section taken along the line D-D in figure 23 of the injection device of the invention after administering the final dose;
- figures 26 and 27 are views to a larger scale of the ringed areas V and X in figure 24;
- figures 28, 29 and 30 are views to a larger scale of the ringed areas W, Y and Z in figure 25;
- figure 31 is a perspective view of the housing of the injection device of the invention;
- figure 32 is a perspective view of a plunger of the injection device of the invention;
- figures 33 and 34 are perspective views of the rear and the front, respectively, of a dosing ring of the injection device of the invention;
- figure 35 is a perspective view of a bush of the injection device of the invention;
- figure 36 is a view analogous to that of figure 35, with the bush turned 180°;
- figure 37 is a perspective view of an actuator of the injection device of the invention;
- figure 38 is a perspective view of a stop member of the injection device of the invention enabling permanent selection of the volume of the dose of medication liquid to be administered;
- figure 39 is a partial perspective view showing an interior wall of a tubular housing and an abutment that co-operates with the bush of the injection device of the invention to set permanently the volume of the dose to be administered; and
- figure 40 is a detail view to a larger scale showing the immobilisation of the bush by means of two detents and a tooth before a first use of the injection device of the invention.

### Detailed description of the preferred embodiments

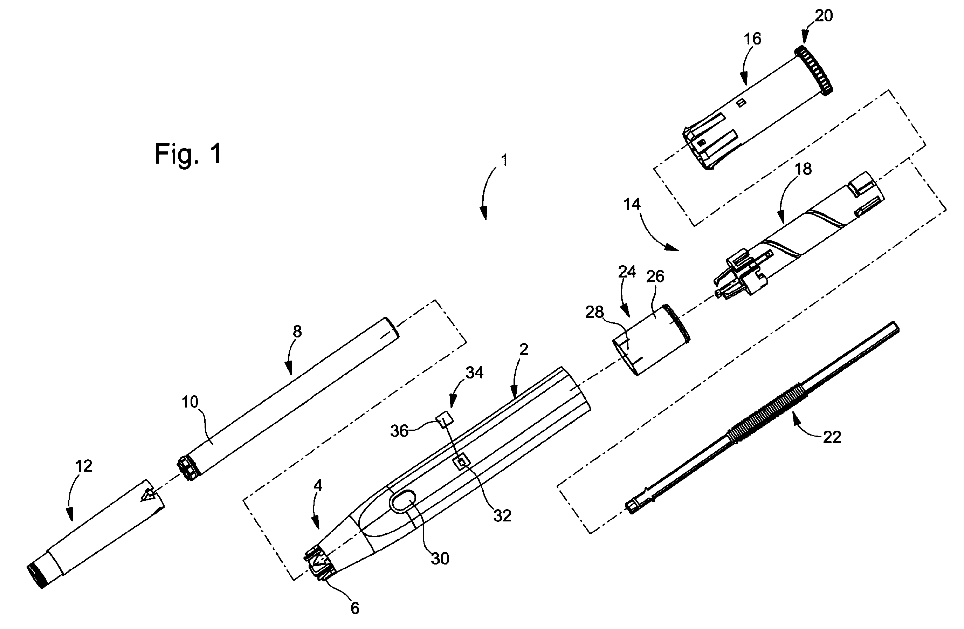

Figure 1 is an exploded perspective view of an injection device of the invention. The injection device 1 comprises a synthetic material tubular housing 2 preferably adapted to be held by a user. The housing 2 has at a distal end 4 retaining means 6 for fixing a cartridge 8 containing a medication liquid to be administered. When shipped, the front end 10 of the cartridge 8 through which the liquid passes when doses of medication are administered is capped by a non-removable cap 12 whose essential function is to protect the cartridge 8 of the injection device 1. The needle, which is not shown in the exploded view, is mounted on a plastics material needle-carrier and clips onto a screwthread at the end of the cap 12, as described in detail hereinafter.

The injection device 1 of the invention also includes an administration mechanism 14 that comprises an actuator 16 and a bush 18 mounted coaxially inside the tubular housing 2. One end 20 of the actuator 16 protrudes from the housing 2 and can be actuated by the user to drive forward a plunger 22 that co-operates with the cartridge 8 to administer doses of medication liquid in the manner described in more detail hereinafter.

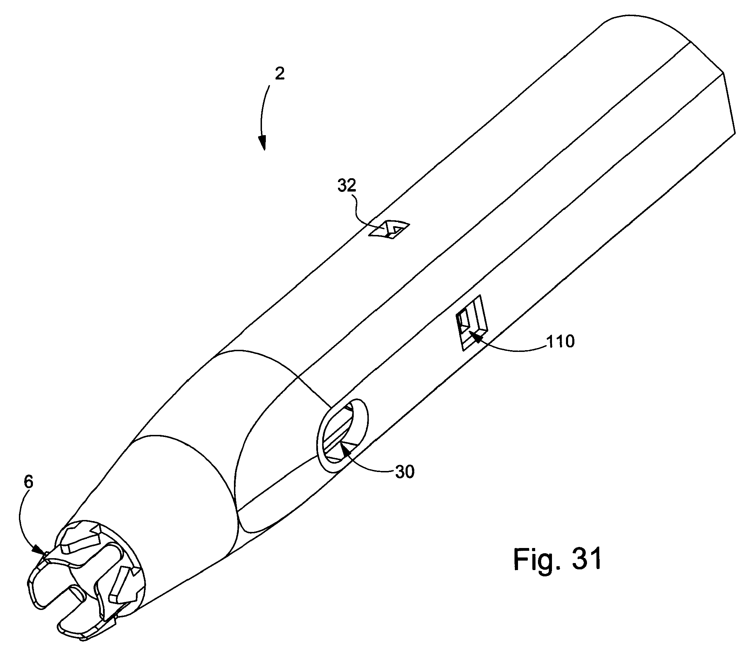

Finally, the injection device 1 of the invention comprises preselector means 24 for preselecting the dose to be administered. In the example shown in the drawing, the preselector means 24 take the form of a dosing ring 26 carrying a dosing scale 28 that is visible from the outside through an observation window 30 in the housing 2 of said injection device 1 (see figure 31).

Note also an opening 32 in the housing 2 of the injection device 1 into which is inserted a stop member 34 in the form of a tooth 36 that is adapted to co-operate with the bush 18 for permanently setting the volume of the dose to be administered.

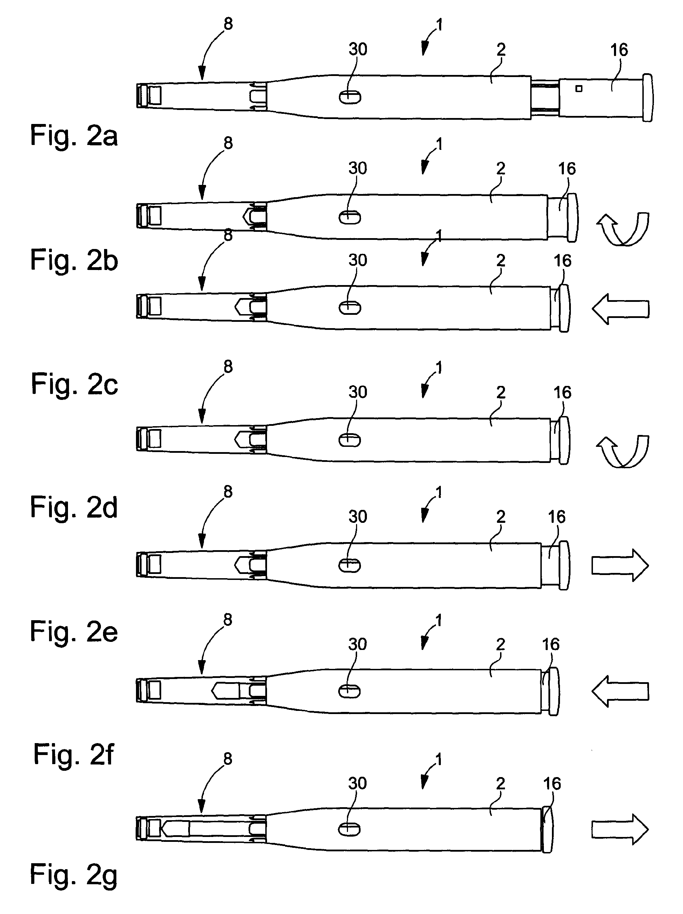

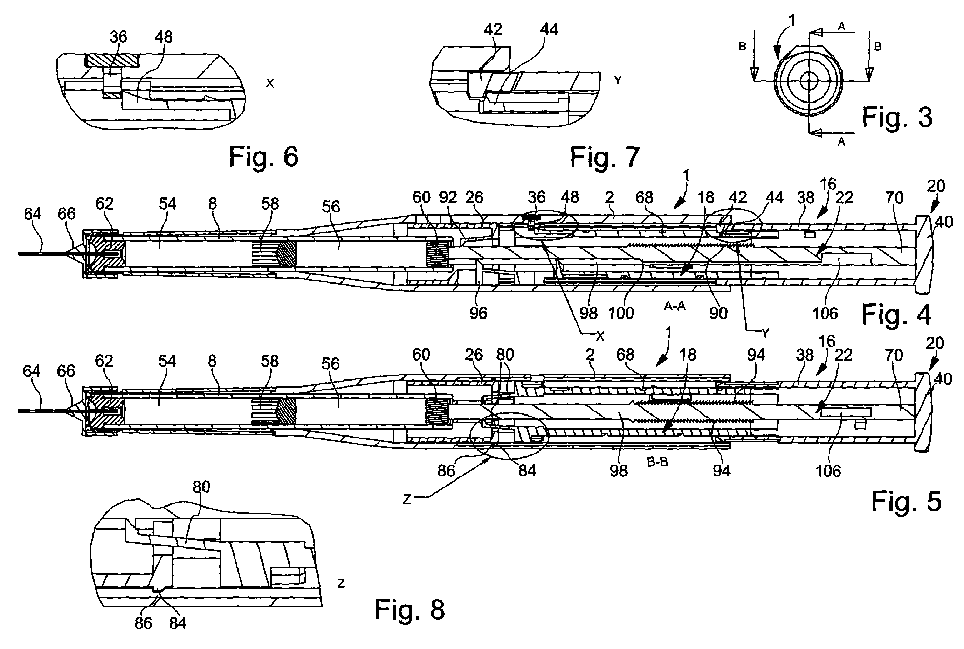

Figure 3 is a front view of the injection device 1 of the invention and figures 4 and 5 are views in section taken along the lines A-A and B-B in figure 2, respectively. Finally, figures 6, 7 and 8 are detail views to a larger scale of the ringed areas in figures 4 and 5.

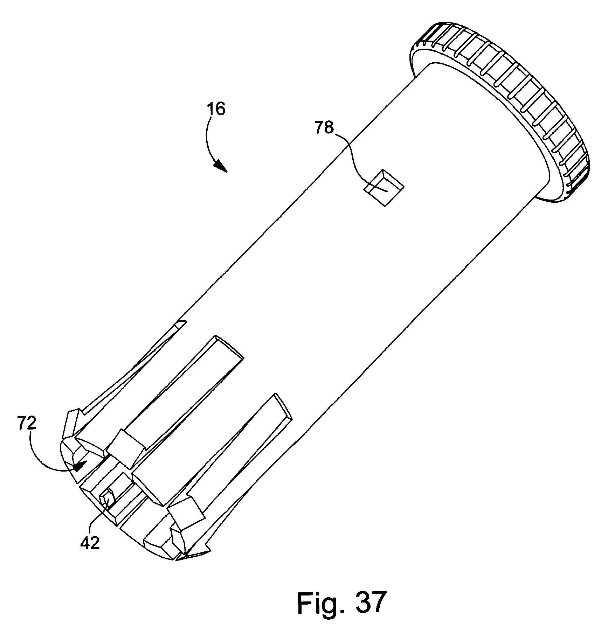

Figures 4 and 5 show the injection device 1 as shipped to the user, before its first use. In this initial configuration, practically all of the actuator 16, which comprises a tubular housing 38 terminated at its end 20 by a button 40, protrudes from the housing 2. Free to move in rotation and in translation in the forward direction inside the housing 2, the actuator 16 is constrained to move in traction with the bush 18 by a pawl 42 that bears against a corresponding abutment 44 on said bush 18 (see figures 4, 7, 35 and 37).

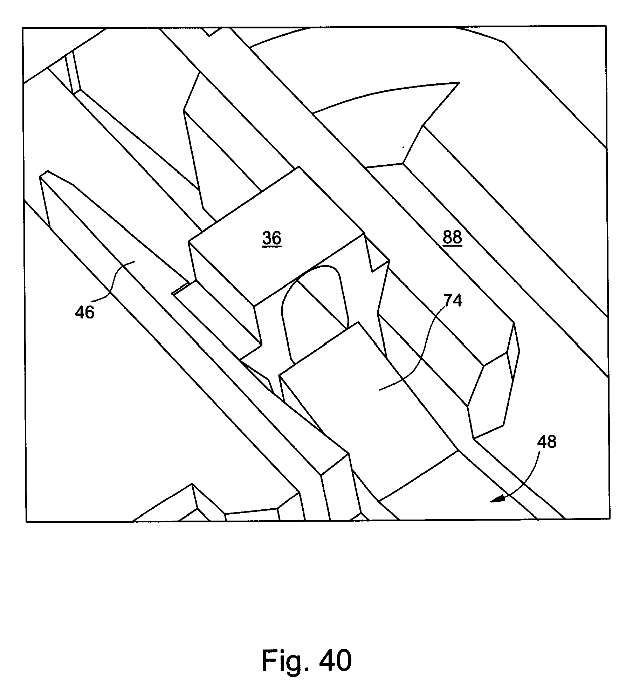

The bush 18 comprises first and second latching means in the form of two detents 46 and 48 that co-operate with the tooth 36, before the first use of the injection device, to immobilise said bush 18 against rotation and movement in translation, respectively (see figures 4, 6 and 40). The housing 2, the bush 18 and the actuator 16 thus form a non-demountable assembly.

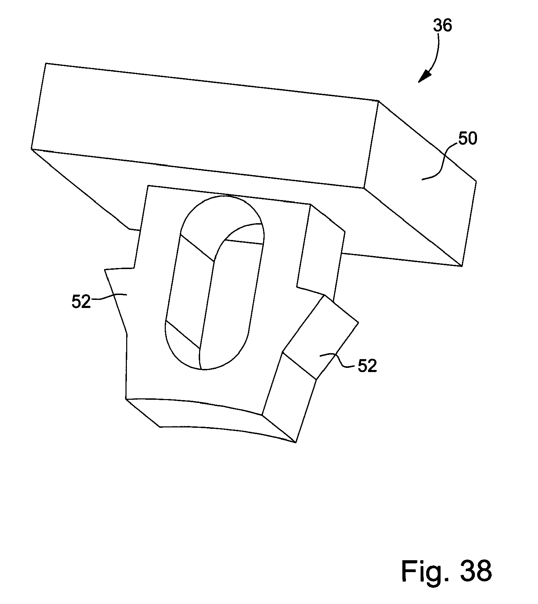

To assemble the injection device 1, the tooth 36 is first inserted through the opening 32. The bush 18 is then inserted axially into the interior space of the tubular housing 2, until its detents 46 and 48 are vertically aligned with the opening 32 and clip onto the tooth 36 to define the axial position of said bush 18. To this end, the tooth 36 comprises a base 50 and two inclined planes 52 on respective opposite sides of its axis of longitudinal symmetry and which diverge in the direction of insertion of said tooth 36 into the opening 32 (see figure 38). The distance between the two inclined planes 52 at their apex is greater than the dimensions of the opening 32. The tooth 36 is therefore forcibly fitted into said opening 32, the inclined planes 52 deforming elastically on passing through the opening 32 and then resuming their initial shape, whereby said tooth 36 is clipped to the housing 2 of the injection device 1.

The injection device 1 is preferably intended for administering doses of a medication liquid that has to be reconstituted prior to use, although this is not limiting on the invention. This applies to growth hormones in particular, which are in a powder state prior to use and must be dissolved in a solvent in order to be injected. This is particularly advantageous in that, in the powder state, growth hormones have a shelf life of the order of five years whereas, once mixed with the solvent, they must be administered within thirty days.

To this end, the volume of the cartridge 8 is divided into two compartments 54 and 56 separated by another movable wall 58, the first of the two compartments 54 containing the active ingredient and the second compartment 56 containing the solvent. One end of the cartridge 8 is closed by the wall 60 forming a seal and movable toward the other end of the cartridge 8, which is closed by a stopper 62 of the septum type and is adapted to be perforated. A needle 64 through which the liquid passes extends through the stopper 62 and is retained thereon by a removable needle-carrier 66. For a complete description of the cartridge, see US Patent 5,716,338, the content of which is hereby incorporated herein by way of reference.

To reconstitute the medication liquid, it is therefore necessary to move the wall 60 forward, which progressively dispels the solvent from the compartment 56 and causes it to enter the compartment 54, where it mixes with the active ingredient. The liquid flows from the chamber 54 to the chamber 56 via grooves in the interior wall of the cartridge 8 level with the wall 58. To this end, it is sufficient for the user to rotate the actuator 16 (see figure 2B), because the pawl 42 on the actuator co-operates with a helical exterior screwthread 68 on the bush 18 (see figures 3, 35 and 36). Accordingly, as it rotates about said bush 18, the actuator 16 progressively enters the housing 2 of the injection device 1, acting on a rear portion 70 of the plunger 22 and causing it to move forward. In turn, the plunger 22 moves the wall 60 of the cartridge 8 forward until it comes into contact with the membrane 58. This transfers the solvent from one compartment to the other and reconstitutes the medication liquid.

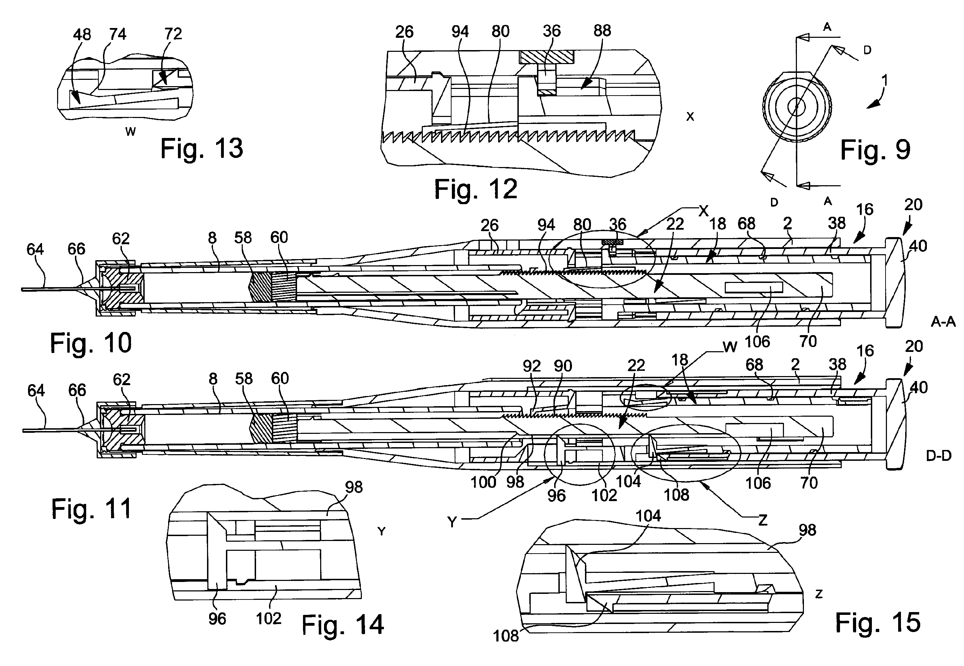

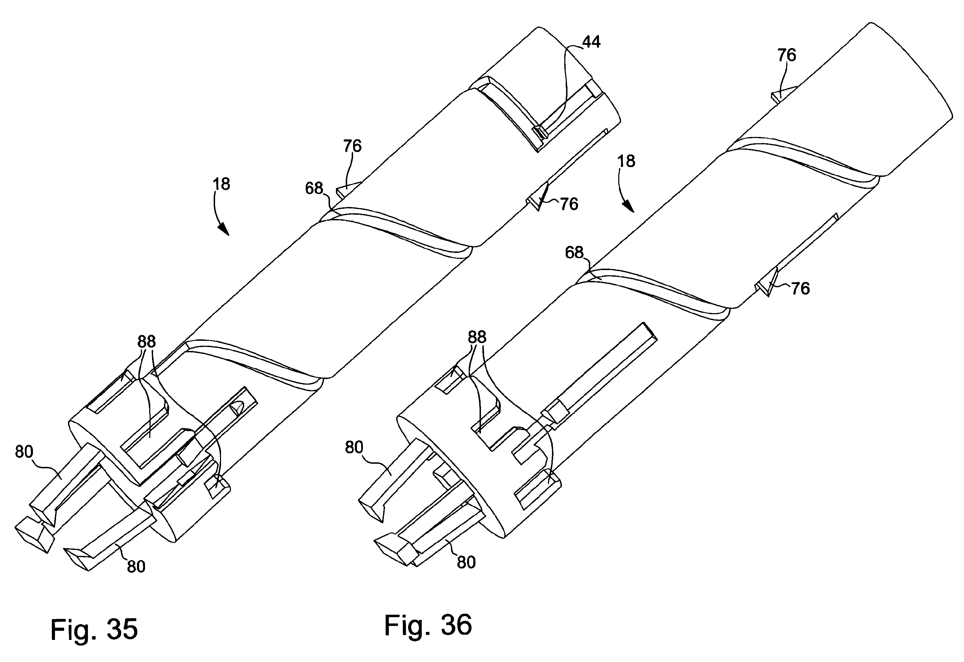

During its forward movement relative to the bush 18, the distal circular rim 72 of the actuator 16 deflects the detent 48 downward, which causes it to escape from the tooth 36 (see figures 11 and 13). This is facilitated by an inclined plane 74 on the detent 48 that restores to the bush 18 its freedom to move forward in translation. Moreover, the bush 18 has at least one tooth on its exterior surface, preferably two teeth 76 that enter two corresponding openings 78 in the housing 38 of the actuator 16 when the pawl 42 of said actuator 16 reaches the end of the helical screwthread 68 (see figures 35, 36 and 37). This immobilises the bush 18 relative to the actuator 16.

When the medication liquid has been reconstituted, it remains to purge the cartridge 8 of any air that it might contain (see figure 2C). To this end, it is sufficient for the user to apply pressure to the button 40 of the actuator 16. This pressure moves the actuator 16 axially forward inside the housing 2 of the injection device 1 and concomitantly moves forward the plunger 22. In turn, the plunger 22 causes the wall 60 and the membrane 58 of the cartridge 8 to move forward, thereby expelling any air trapped in said cartridge 8. The forward movement of the actuator 16 stops when the bush 18 abuts against the dosing ring 26.

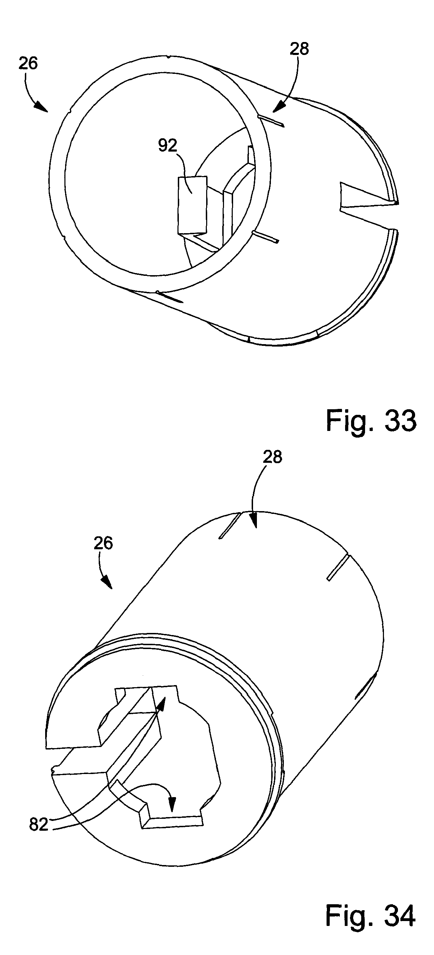

From this latter position, the user can set the volume of the dose to be injected. The actuator 16 must be rotated (see figure 2D). Because the bush 18 is immobilised relative to said actuator 16, it also turns, entraining the dosing ring 26 as it rotates. The bush 18 comprises engagement means in the form of at least one ratchet, preferably two ratchets 80 whose function is described hereinafter and which project into the interior volume of the dosing ring 26, sliding in two complementary shape grooves 82 in said dosing ring 26 (see figure 34). Thus the dosing ring 26, which is immobilised axially by way of a peripheral shoulder 84 that engages in a circular groove 86 in the internal wall of the housing 2 of the injection device 1, is entrained in rotation by the bush 18, and thus by the actuator 16. The user therefore sees the marks on the dosing scale 28 move across the observation window 30, and stops rotating the actuator 16 when the reading corresponds to the volume of the prescribed dose. Advantageously, before setting the dose, it is possible to rotate the actuator to view all doses if so is desired.

From this latter position, the user must pull back on the actuator 16 (see figure 2E). As the bush 18 is fastened to the actuator 16, it is also pulled back. On the other hand, the dosing ring 26 is constrained to move in translation with the housing 2 of the injection device 1 and therefore remains immobile. Because of this rearward movement, the tooth 36 enters one of the peripheral longitudinal grooves 88 in the bush 18 (see figures 35 and 36). The number of longitudinal grooves, which are all different lengths, is equal to the number of different volumes that the injection device 1 is able to dispense. In the present example there are six grooves 88, which means that it is possible to administer to oneself or to another person doses comprising one of six volumes. This example is purely illustrative, of course. Thus, when a mark on the dosing scale 28 is in the observation window 30, the tooth 36 is lined up with whichever of the grooves 88 corresponds to that mark. Then, pulling on the actuator 16 causes the tooth 36 to enter the selected groove 88. Its movement stops when said tooth 36 reaches the bottom of said groove 88. The tooth 36 therefore defines with the groove 88 in which it slides a position in which the bush 18, and therefore the actuator 16, is pulled back as far as possible, said bush 18 being also immobilised in the forward direction by the dosing ring 26. The tooth 36 and the dosing ring 26 thus define the usable travel of the bush 18 within the housing 2 of the injection device 1 and therefore determine the volume of the dose to be injected.

Figure 9 is a front view of the injection device 1 according to the invention and figures 10 and 11 are views in section taken along the lines A-A and D-D, respectively, in figure 9. Finally, figures 12 to 15 are detail views to a larger scale of the ringed areas in figures 10 and 11.

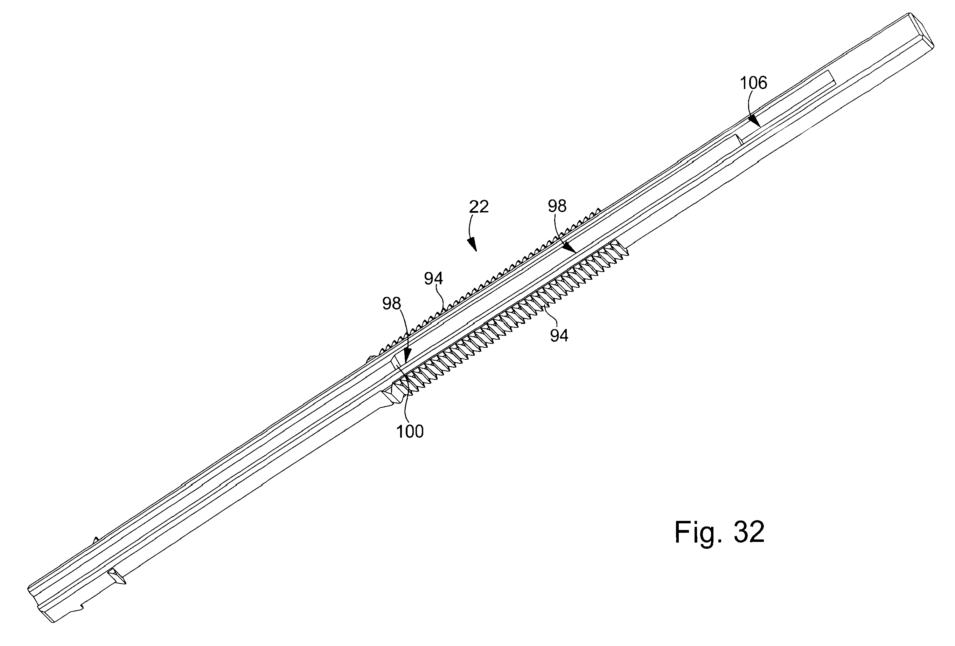

It is important to note that the plunger does not move during the rearward movement of the bush 18. The plunger 22 comprises a first rack 90 through which it co-operates with a ratchet 92 of the detent type on the dosing ring 26 (see figure 33). The ratchet 92 immobilises the rack 90 and therefore the plunger 22 on movement in the rearward direction and escapes from the rack 90 on movement in the forward direction. Moreover, the plunger 22 comprises two further racks 94 (see figure 32) which co-operate with the ratchets 80 of the bush 18. The ratchets 80 escape from the racks 94 on movement of said bush 18 in the rearward direction and push on the racks 94 on movement in the forward direction, causing the plunger 22 to move forward. Said plunger 22 has a square or rectangular section, with the two racks 94 on two opposite faces of the plunger 22 and the rack 90 on one of the remaining two faces.

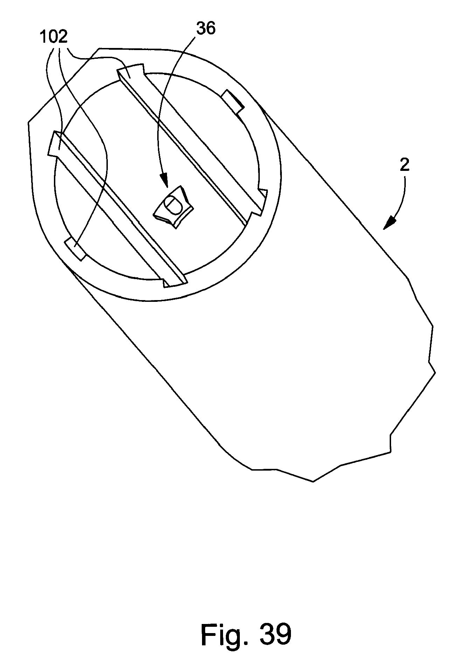

The bush 18 also comprises an immobilising member 96 of the detent type that co-operates with a cam surface 98 on the plunger 22. When the user pulls on the actuator 16, and therefore on the bush 18, to arm the injection device 1 and to place it in a condition ready to deliver the first dose of the medication liquid, the latch 96 moves along the cam surface 98 and passes over a step 100, which causes it to enter one of the facing grooves 102 formed in the interior wall of the tubular housing 2 (see figure 39). Regardless of subsequent movements of the bush 18 relative to the plunger 22, the latch 96 does not leave the groove 102 into which it projects, thereby immobilising the bush 18 and thus likewise the dosing ring 26 against rotation. Accordingly, the dosing ring 26 is immobilised permanently after setting the volume of the dose to be administered. It will be noted that the number of grooves 102 is the same as the number of peripheral grooves 88 on the bush 18 and that the former grooves are in corresponding relationship to the latter grooves.

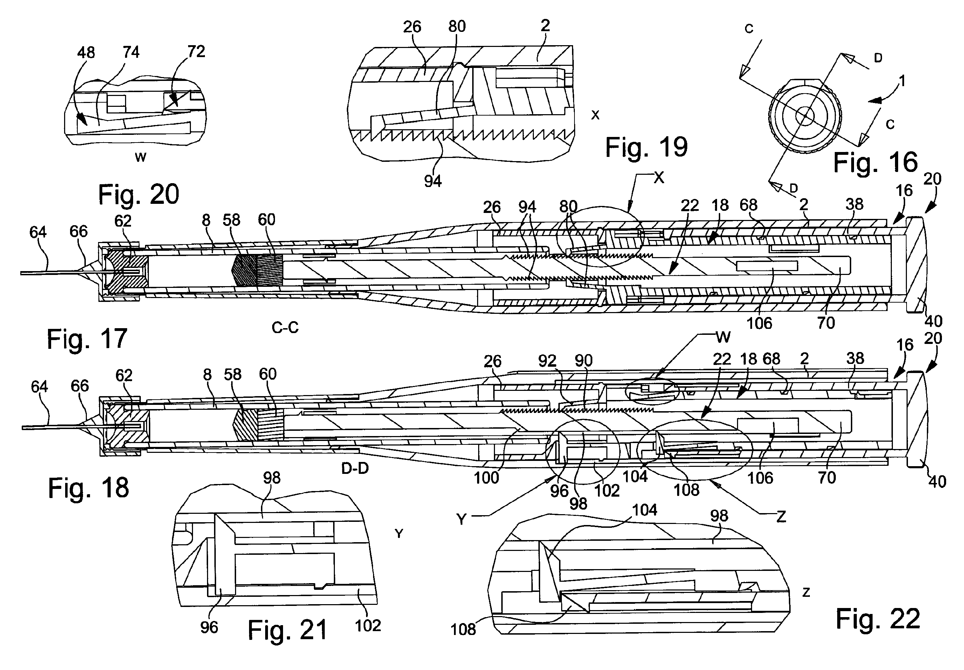

Figure 16 is a front view of the injection device 1 of the invention and figures 17 and 18 are views in section taken along the lines C-C and D-D in figure 16. Finally, figures 19 to 22 are detail views to a larger scale of the ringed areas in figures 17 and 18.

When the user has armed the injection device 1 by pulling on the actuator 16, as shown in figures 10 and 11, it remains only to administer the injection. To this end, he must first insert the needle 64 into the skin and then press on the actuator 16 (see figure 2F). This pressure moves said actuator 16, and therefore the bush 18, axially inside the housing 2 of the injection device 1. As it moves forward, the bush 18 causes the plunger 22 to move forward by virtue of the ratchets 80 pushing on the racks 94 on said plunger 22. Finally, the plunger 22 pushes the wall 60 and the wall 58 forward, the effect of which is to expel a dose of medication liquid from the cartridge 8. The movement stops when the bush abuts against the dosing ring 26.

To rearm the injection device 1, it is sufficient to pull on the actuator 16 again. The rearward movement stops when the tooth 36 reaches the end of the groove 88 in which it slides (see figure 2G). It will be noted that the plunger 22 does not move during this rearward movement, because it is held in place by the ratchet 92 engaged with the rack 90, whereas the ratchets 80 escape from the racks 94. In other words, the plunger 22 moves forward when the user presses on the actuator 16 and does not move when the user pulls on the actuator 16. The plunger 22 therefore moves only forward inside the tubular housing 2 and never backward.

Once rearmed, the injection device 1 is ready to deliver a new dose of medication liquid. This cycle repeats up to dispensing the final dose.

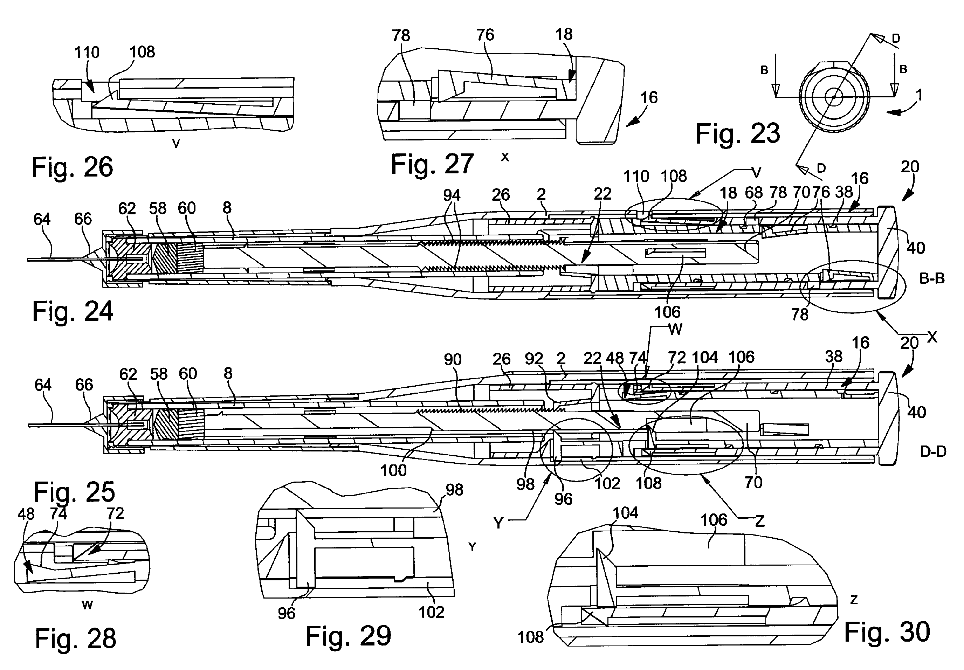

What happens on dispensing the final dose of medication liquid is described next with reference to figures 23 to 30. Figure 23 is a front view of the injection device of the invention and figures 24 and 25 are views in section taken along the lines B-B and D-D, respectively, in figure 23. Finally, figures 26 to 30 are detail views to a larger scale of the ringed areas in figures 24 and 25.

The bush 18 comprises a latching member 104 in the form of a detent that co-operates with the cam surface 98 and drops into a cavity 106 when the plunger 22 reaches its end of travel position. By dropping into the cavity 106, the detent 104 is retracted from in front of the actuator 16, which is therefore allowed to move forward. Thereafter, the connection via the teeth 76 and the openings 78 between the actuator 16 and the bush 18, which is activated as soon as reconstitution ends, becomes inoperative when the actuator 16 moves forward relative to the bush 18. During this movement, an immobilising member 108 of the latch type carried by the actuator 16 projects into an opening 110 formed in the housing 2 of the injection device 1 to immobilise the injection device permanently after dispensing the final dose. The actuator 16 is locked to the housing 2 of the injection device 1, while the bush 18 is immobilised in translation in the forward direction by the dosing ring 26 and in the rearward direction by the internal surface of said actuator 16 with which said bush 18 comes into contact. The other end of the cartridge 8 is mounted in a non-demountable manner on said housing 2. Thus the injection device 1 according to the invention cannot be demounted or re-used after administering the final dose of medication liquid, and may be discarded.

It goes without saying that the present invention is not limited to the embodiment that has just been described and various simple variants and modifications may be envisaged by the person skilled in the art that do not depart from the scope of the present invention.

## Claims

1. An injection device for administering multiple doses of a medication liquid, said device (1) comprising a housing (2), a cartridge (8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that** it comprises means for permanently setting the dose to be administred.

2. An injection device for administering multiple doses of a medication liquid, said device (1) comprising a housing (2), a cartridge(8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that** it comprises means for permanently immobilising the preselector means (24) after setting the dose to be administered.

3. An injection device according to claim 1 or 2, **characterised in that** the plunger (22) has along its length a cam surface (98) which co-operates with at least one immobilising member that is latched when the plunger (22) reaches a predefined position.

4. An injection device according to claim 3, **characterised in that** the immobilising member is a detent (96) which is latched when it enters a longitudinal groove (102) in the housing (2) of said injection device (1) .

5. An injection device according to any one of claims 1 to 4, **characterised in that** it further comprises a latching member that co-operates with the cam surface (98) and is latched when the plunger (22) reaches a predetermined position to prevent extraction of the administration mechanism (14) after administering the final dose.

6. An injection device according to claim 5, **characterised in that** the latching member is a detent (104) which when latched is retracted from in front of an immobilising member (108) that projects into an opening (110) in the housing (2) of said injection device (1) to immobilise the administration mechanism (14) permanently after dispensing the final dose.

7. An injection device for administering multiple doses of a medication liquid, said device (1) comprising a housing (2), a cartridge(8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that** it comprises means for permanently fastening the actuator (16) to the housing (2) after administering the final dose of the medication liquid.

8. A device according to claim 7, **characterised in that** the bush (18) is constrained to move with the actuator (16) in rotation and in translation during a first use of said injection device (1), the bush (18) comprising a latching member (104) adapted to co-operate with a cam surface (98) on the plunger (22), and said latching member (104) being retracted when the bush (18) reaches a predetermined position relative to said plunger (22) to allow forward movement of the actuator (16), a locking member of which projects into an opening (110) in the housing (2) of said injection device (1) to lock the latter permanently after dispensing the final dose.

9. An injection device according to claim 8, **characterised in that** the bush (18) comprises at least one tooth (76) that enters a corresponding opening (78) in the actuator (16) to immobilise it temporarily relative to said actuator (16).

10. An injection device for administering multiple doses of a medication liquid, said device (1) comprising a housing (2), a cartridge(8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that** it further comprises a bush (18) adapted to be moved forward and backward within the housing (2) and having at its periphery longitudinal grooves (88) of different lengths, a first stop member (34) being adapted to enter any of said grooves (88) and to define with said groove a position of maximum retraction of the bush (18), said bush (18) being also adapted to be immobilised in the forward direction by a second stop member that defines with the first stop member the active travel of the bush (18) within the housing (2) and thereby determines the volume of the dose to be injected.

11. An injection device according to claim 10, **characterised in that** the first stop member (34) comprises a tooth (36) that is inserted through an opening (32) formed in the housing (2) of the injection device (1) and is fastened to said housing (2).

12. An injection device according to either claim 10 or claim 11, **characterised in that** the preselector means (24) for preselecting the dose to be injected form the second bush stop member.

13. An injection device according to any of claims 10 to 12, **characterised in that** the preselector means (24) for preselecting the dose to be injected comprise a dosing ring (26) fixed in translation inside the device (1) but free to rotate before selecting the dose to be injected, said dosing ring (26) carrying a dosing scale (28) that is visible from the outside through an observation window (30) in the housing (2) of said injection device (1) and said dosing ring (26) being constrained to rotate with the bush (18), both of which are turned until the first stop member (34) engages in one of the grooves (88) of said bush (18).

14. An injection device according to claim 13, **characterised in that** the bush (18) further comprises an immobilising member that co-operates with a cam surface (98) on the plunger (22) and is latched when the bush (18) reaches a predetermined position relative to said plunger (22), the latching member entering a longitudinal groove (102) in the housing (2) to prevent rotation of the bush (18) and therefore of the dosing ring (26).

15. An injection device for administering multiple doses of a medication liquid, said device comprising a housing (2) adapted to be held by a user and to a distal end of which is fixed an cartridge (8) containing the liquid to be administered, said cartridge (8) having one end closed by a movable wall (60) that is adapted to be moved toward the other end of the cartridge (8), which is closed by a stopper (62) adapted to be perforated and through which the liquid passes, and an administration mechanism (14) for actuating the wall (60) via a plunger (22) whose forward movement inside the housing (2) is driven by an actuator (16) at the proximal end of the housing (2) and adapted to be actuated by the user, the injection device (1) further comprising preselector means (24) for preselecting the dose to be injected, and said injection device (1) being **characterised in that** the plunger (22) has along its length a cam surface (98) which co-operates with at least one latching member that is latched when the plunger (22) reaches a predefined position.

16. An injection device according to claim 15, **characterised in that** it comprises a latching member that co-operates with the cam surface (98) and is latched when the plunger (22) reaches a predefined position to immobilise permanently the preselector means (24) after setting the dose to be administered.

17. An injection device according to claim 16, **characterised in that** the latching member is a detent (96) which is latched when it enters a longitudinal groove (102) in the housing (2) of said injection device (1).

18. An injection device according to any of claims 15 to 17, **characterised in that** the latching member is a detent (104) which, when latched, is retracted in front of an immobilising member (108) that projects into an opening (110) in the housing (2) of said injection device (1) to immobilise the administration mechanism (14) permanently after dispensing the final dose.

19. An injection device for administering multiple doses of a medication liquid, said device (1) comprising a housing (2), a cartridge(8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that**, in a first mode of operation for reconstituting the medication liquid, the plunger (22) is actuated by the actuator (16), which operates on the rear portion (70) thereof, and **in that**, in a second mode of operation for dispensing the medication liquid, the actuator (16) operates on the plunger (22) via a system of detents and racks.

20. An injection device according to claim 19, **characterised in that** the plunger (22) comprises at least one rack (94) and the actuator (16) co-operates with a bush (18) that comprises ratchet means (80) that escape from the rack (94) on movement in the rearward direction and which push on the rack (94) on movement in the forward direction, the actuator (16) being fastened to the bush (18) only in traction before the first use and being fastened to said bush (18) to rotate and move in translation therewith after reconstituting the medication liquid, whereby during reconstitution of the medication liquid the actuator (16), when actuated by the user, moves forward and pushes on the plunger (22) which in turn pushes forward the wall (60) of the cartridge (8), during preparation of a dose, the actuator (16), fastened to the bush (18), is retracted, the plunger (22) remaining immobile, and, during administration of a dose, the actuator (16), when actuated by the user, moves forward and pushes forward the plunger (22) via the ratchet means (80).

21. An injection device according to claim 20, **characterised in that** the plunger (22) comprises a second rack (90) on which act ratchet means (92) that comprise the preselector means (24) for preselecting the dose to be injected, said ratchet means (82) immobilising the rack (90) on movement in the rearward direction and escaping from the rack (90) on movement in the forward direction.

22. An injection device for administering multiple doses of a medication liquid, said device (1) comprising a housing (2) a cartridge(8) containing a preparation or precursor component for the preparation and having a front part and a rear part, a stopper (62) arranged at the front part and the rear part being closed by a movable wall (60), a displacement of which wall causes the preparation to be moved forward or expelled through the stopper adapted to be perforated, for the preparation, and actuating means (14) for actuating the wall (60) via a plunger (22) whose forward movement inside the housing is driven by an actuator (16) at the proximal end of the housing (2) and adapted to be actuated by the user, the injection device (1) further comprising preselector means (24) for preselecting a dose to be injected and said injection device (1) being **characterised in that** it comprises a bush (18) comprising first and second latching means (46, 48) that co-operate with a stop member (34) to immobilise said bush (18) against rotation and against movement in translation, respectively, the actuator (16) and the bush (18) being such that, on a first use, the actuator (16), when actuated by the user, moves forward relative to said bush (18), which remains fixed and releases the second latching means (48), with the result that the bush (18) is free to move in translation, said actuator (16) then being fixed to said bush (18) to move in translation and to rotate therewith.

23. An injection device according to claim 22, **characterised in that** the bush (18) comprises an external helical screwthread (68) within which is engaged a pawl (42) on the actuator (16), whereby rotation of the actuator (16) relative to said bush (18) causes it to enter the housing (2) of said injection device (1).

24. An injection device according to claim 23, **characterised in that**, before the first use of the device, the bush (18) is fastened to the actuator (16) only in traction.

25. An injection device according to claims 23 and 24, **characterised in that** the bush (18) is fastened to the actuator (16) in traction by its pawl (42), which bears against an abutment (44) at a proximal end of the helical screwthread (68).
